# EUROPEAN PATENT APPLICATION

(11) **EP 4 218 638 A1**
(43) Date of publication of application: **02.08.2023**
(21) Application number: 23150153.7
(22) Date of filing: 03.01.2023
(51) Int. Cl.: A61B 18/02, A61B 90/00

(54) **METHOD FOR MANAGING REFRIGERANT PRESSURE FOR CRYOABLATION AND CRYOMAPPING**

(30) Priority: 26.01.2022 US 202263303215 P
(71) Applicant: Medtronic, Inc., Minneapolis, Minnesota 55432 (US)
(72) Inventor: MA, Wing-Cho, Minneapolis, 55432 (US); RUCHTI, Nicholas, Minneapolis, 55432 (US); NAGEL, Nicholas L., Minneapolis, 55432 (US); ROCHON, Martin, Minneapolis, 55432 (US); DREIER, Daniel T., Minneapolis, 55432 (US); LEY, Cary M., Minneapolis, 55432 (US); SCHRAUT, Julia A., Minneapolis, 55432 (US)
(74) Representative: Zimmermann & Partner Patentanwälte mbB

(57) **Abstract**

A cryoablation system, cryoablation console and method for managing pressure of a pressurized refrigerant are disclosed. According to one aspect, a method includes heating the pressurized refrigerant in at least one of a reservoir and a tank to increase a pressure of the pressurized refrigerant toward a target pressure. The method further includes delivering the heated pressurized refrigerant to a cryoablation catheter.

## Description

### FIELD

The present technology is generally related to managing refrigerant pressure for cryoablation and cryomapping.

### BACKGROUND

Catheter Cryoablation is a technique that may be used to ablate tissue and has applications in, for example, cancer, nerve and cardiac treatment. For example, cryoablation may be used to control heart rhythm by ablating certain tissues that cause abnormal heart rhythms. In cryoablation, the ablation catheter may be used to create lesions where heat is rapidly removed from cardiac cells, by delivering pressurized refrigerant, such as nitrous oxide ("N2O"), with a controlled mass flow rate, to a cryoablation catheter. Heat may be transferred as the pressurized refrigerant expands and evaporates in the catheter tip. Cardiac cell lesions may be created via the rapid removal of heat.

Catheter Cryomapping is a focal catheter technique that may be used to reversibly impair an action potential of cardiac cells without creating lesions. The purpose of this technique is to identify the effectiveness of potential locations to apply cryoablation prior to creating a lesion. Cryomapping is like cryoablation in that the catheter is placed at the target site where heat is rapidly removed from cardiac cells by the delivering of pressurized refrigerant, such as nitrous oxide ("N2O"). Where cryomapping differs from cryoablation is that it is controlled by monitoring the targeted catheter tip temperature and adjusting the refrigerant pressure to maintain that target temperature.

A console is provided to control the delivery of the pressurized refrigerant. During cryomapping, the console may monitor the cryoablation catheter's temperature and control the delivery of pressurized refrigerant to the tip of the cryoablation catheter, such as a focal catheter, to try and maintain a target temperature. Clinically, the temperature during cryomapping is set so that the effect the refrigerant has on the cardiac cell tissue may be reversible. The console has a tank which may be able to hold the pressurized refrigerant. The refrigerant needs to be under high pressure. The pressure of the refrigerant in the tank may be proportional to the temperature of the refrigerant in the tank.

The pressure versus temperature relationship is governed by thermodynamic principles. The pressurized refrigerant must be under high pressure (eg. 760 psig) to remain in liquid form until it reaches the tip of the cryoablation catheter to deliver the cryotherapy, where a pressure change occurs from high pressure to less than atmospheric pressure leading to a phase change from liquid to vapor. This thermodynamic process absorbs heat from the surrounding tissues of the catheter tip and ablates the tissues. In a cold room or with a cold pressurized refrigerant source, the pressure of the refrigerant may not be high enough to perform optimal cryotherapy.

Thus, at times, the environmental conditions around the console and tank may cause the pressure of the refrigerant to be low or high. When the pressure of the refrigerant is low, a longer time is required to for the pressure of the refrigerant to rise to the desired level, leading to inconsistent therapies. Low pressure may cause performance issues within the console as well. These performance issues may include inability to meet a target mass flow rate for cryoablation and inability to maintain a target catheter temperature for cryomapping.

### SUMMARY

The techniques of this disclosure generally relate to managing refrigerant pressure for cryoablation and cryomapping.

Further disclosed herein is a cryoablation system, a cryoablation console and a method for managing pressure of a pressurized refrigerant are disclosed, wherein, according to one aspect, a method includes heating the pressurized refrigerant in at least one of a reservoir and a tank to increase a pressure of the pressurized refrigerant toward a target pressure, wherein the method further includes delivering the heated pressurized refrigerant to a cryoablation catheter.

The details of one or more aspects of the disclosure are set forth in the accompanying drawings and the description below. Other features, objects, and advantages of the techniques described in this disclosure will be apparent from the description and drawings, and from the claims.

### BRIEF DESCRIPTION OF THE DRAWINGS

Some embodiments will be better understood by reference to the following detailed description when considered in conjunction with the accompanying drawings wherein:
FIG. 1 illustrates a cryoablation system including a pressurized refrigerant controller constructed according to principles set forth herein;
FIG. 2 illustrates a pressure control loop configured to control a pressure of a pressurized refrigerant source and constructed according to principles set forth herein;
FIG. 3 is a flowchart of an example process for managing refrigerant pressure of a pressurized refrigerant source according to principles set forth herein; and
FIG. 4 is a flowchart of another example process for managing refrigerant pressure of a pressurized refrigerant source according to principles set forth herein.

### DETAILED DESCRIPTION

Methods and systems for managing refrigerant pressure for cryoablation and cryomapping in a cryoablation system are disclosed. In a cold environment, or with a tank of pressurized refrigerant stored in a cold environment, the pressure of the refrigerant in the tank may not be high enough to achieve cryotherapy, and a long warm uptime is needed for pressure to rise to a level needed to deliver the cryo-therapy. Some embodiments overcome the problem of latency when bringing pressurized refrigerant up to a desired pressure, by providing a heating element removably affixed to at least one of a reservoir and/or a tank that provides a source of pressurized refrigerant to a cryoablation catheter. The reservoir and/or tank stores pressurized refrigerant. In some embodiments, there is a tank but no reservoir. In some embodiments, there is a reservoir only, the reservoir being fed by a high pressure refrigerant line. In some embodiments, there may be a tank and a reservoir and either one or both may be heated. The reservoir may be smaller than the tank and can be heated more quickly and efficiently than the tank can be heated, in some embodiments. This enables pressure pressurized refrigerant received from the tank to be more quickly and efficiently heated in the reservoir before delivery to the cryoablation catheter. Accordingly, in addition to the reservoir and/or tank, a heater is provided to heat the pressurized refrigerant in the reservoir and/or tank to achieve a target pressure. A controller is provided to adjust a temperature setting of the heater based at least in part on a detected pressure and/or a detected temperature. In some embodiments, a pressure control loop is provided to drive a difference between a target pressure and a calculated pressure or measured pressure toward zero.

Before describing in detail exemplary embodiments, it is noted that the embodiments reside primarily in combinations of apparatus components and processing steps related to feedback control systems for cryo-mapping and cryoablation. Accordingly, components have been represented where appropriate by conventional symbols in the drawings, showing only those specific details that are pertinent to understanding the embodiments so as not to obscure the disclosure with details that will be readily apparent to those of ordinary skill in the art having the benefit of the description herein. Like numbers refer to like elements throughout the description.

It should be understood that various aspects disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques). In addition, while certain aspects of this disclosure are described as being performed by a single module or unit for purposes of clarity, it should be understood that the techniques of this disclosure may be performed by a combination of units or modules associated with, for example, a medical device.

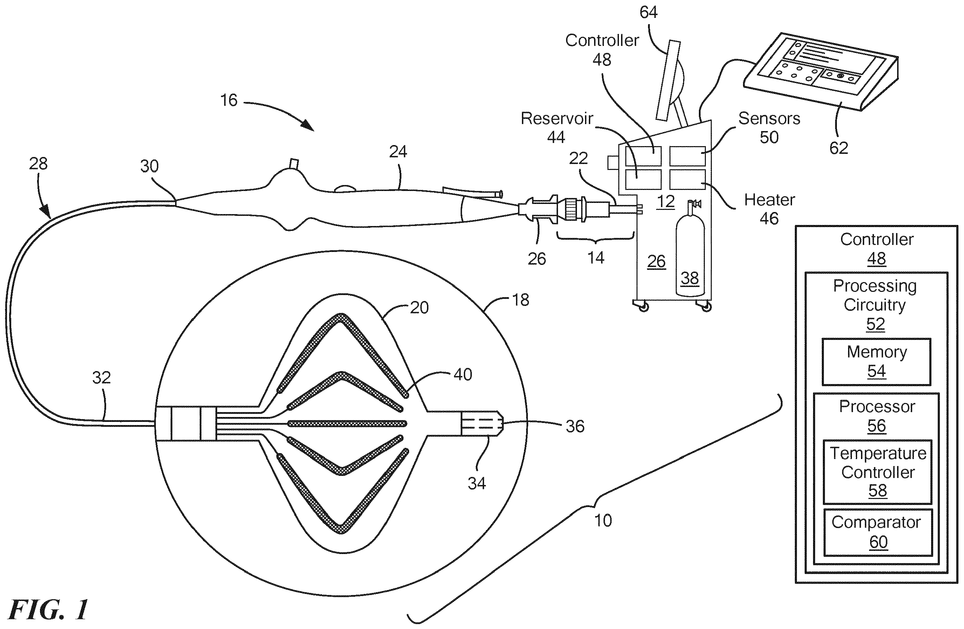

Referring now to the drawing figures where like elements have like reference numerals, FIG. 1 illustrates an exemplary cryoablation system 10 that may be used to map and treat tissue. The cryoablation system 10 may include a cryoablation console 12 coupled to one end of an umbilical system 14. The opposing end of the umbilical system 14 may be coupled with a cryoablation catheter 16. The cryoablation catheter 16 may be any medical device including a medical device with an inflatable portion including a catheter and a balloon catheter or it may be a medical device with a focal catheter. As shown in FIG. 1, the cryoablation catheter 16 may include a treatment portion 18. As shown in FIG. 1, the treatment portion 18 may include an inflatable portion 20. However, the cryoablation catheter 16 may not have an inflatable portion 20 and may have a different type of treatment portion 18 including a focal catheter.

The umbilical system 14 may include a coaxial cable umbilical 22 which includes both a cooling injection umbilical and a vacuum umbilical that provide respective inlet and return paths for pressurized refrigerant used to cool a tissue-treating end of the treatment portion 18. The vacuum umbilical may be used to allow excess pressurized refrigerant or gas to escape from the cryoablation catheter 16 and the cooling injection umbilical may transfer pressurized refrigerant from the cryoablation console 12 to the cryoablation catheter 16.

Continuing to refer to FIG. 1, the cryoablation console 12 may be coupled to the cryoablation catheter 16 via the umbilical system 14 and the cryoablation catheter 16 may include a handle 24 having a coaxial connector 26 with both an injection lumen and vacuum lumen therein. The cryoablation catheter 16 may further include an elongate body 28 having a proximal portion 30 and a distal portion 32 which define a pressurized refrigerant flow path and a distal end 34, the distal end 34 may include a lumen therein. The treatment portion 18 of the cryoablation catheter 16 may be coupled to the elongate body 28. The elongate body 28 may be a flexible tubing that couples pressurized refrigerant from a pressurized refrigerant source such as a tank 38 to the treatment portion 18 and the distal portion 32 of the elongate body 28. For example, cold pressurized refrigerant may be injected into the cooling injection umbilical within the elongate body 28 to the treatment portion 18 of the cryoablation catheter 16. The vacuum umbilical within the elongate body 28 may evacuate the injected pressurized refrigerant from the treatment portion 18 back to the cryoablation console 12.

The treatment portion 18 may have no electrodes or at least one electrode 40 to contact surrounding tissue and sense electrical activity in the tissue in the vicinity of the at least one electrode 40. It will be understood that the at least one electrode 40 may be one or more than one electrode in a variety of different shapes, sizes, and configurations. The cryoablation system 10 may generally include the cryoablation catheter 16 that may be coupled indirectly to a tank 38 or a high pressure line to supply pressurized refrigerant to the cryoablation catheter 16. When a high pressure line provides the pressurized refrigerant, rather than a tank 38, then there may be a reservoir 44 present to receive the pressurized refrigerant, and the reservoir 44 is then heated according to the methods described herein. In some embodiments, the tank 38 acts as a reservoir and/or is coupled to a reservoir 44 which is coupled to the cryoablation catheter 16. Thus, in some embodiments, there may be tank 38 but no reservoir 44, in which case the tank 38 is heated, or there may be a reservoir 44 but no tank 38, in which case the reservoir 44 is heated, or there may be both a tank 38 and a reservoir 44. In those embodiments that include both a tank 38 and a reservoir 44, then heating may be applied to the tank 38 but not the reservoir 44, or heating may be applied to the reservoir 44 but not the tank 38, or heating may be applied to the tank 38 and the reservoir 44. Typically, the tank may be a standalone source of pressurized refrigerant, whereas a reservoir may be a holding tank that receives pressurized refrigerant from another source.

Thus, the pressurized refrigerant in at least one of a reservoir 44 and a tank 38 may be heated by a heater 46 attached directly to the tank 38 and/or reservoir 44 in order to rapidly increase the pressure of the pressurized refrigerant prior to delivery of the pressurized refrigerant to the cryoablation catheter 16. The reservoir 44 may therefore be smaller than the tank 38 to enable more rapid and efficient heating of the pressurized refrigerant than would be enabled by heating the tank 38. The cryoablation console 12 also includes a controller 48 in communication with the tank 38 and/or reservoir 44 and the heater 46. In some embodiments, the controller 48 may be in communication with one or more sensors 50, which may include a thermocouple to sense a temperature of the pressurized refrigerant in the tank 38 and/or reservoir 44, and/or may include a pressure transducer to sense a pressure of the pressurized refrigerant in the tank 38 and/or reservoir 44. In some embodiments, multiple reservoirs 44 may be implemented to enable rapid increase of pressure of the pressurized refrigerant by heating, via one or more heaters 46, one or more of the multiple reservoirs 44. An advantage to having multiple reservoirs 44 is an ability to increase pressure more rapidly by increasing pressurized refrigerant temperature in stages.

Note that in some embodiments, the heater 46 may be configured with overcurrent protection, such as by an electrical fuse, and insulation. The heating element 46 may be provided with a mechanical thermo-protector and be strapped to the tank 38 and/or reservoir 44 to make good contact between a heating element of the heater 46 and the tank 38 and/or reservoir 44. Thus, in some embodiments, a heating element can be removably affixed to one or more of a tank, a reservoir, or multiple reservoirs.

In some embodiments, the heat provided by the heater 46 may be controlled by turning the heater 46 on and off. In some embodiments, the heat provided by the heater 46 may be adjusted by degrees. In some embodiments, the temperature or heat provided by the heater 46 may be limited to avoid compromise of the integrity of the tank 38 and/or the reservoir 44. In some embodiments, the heater 46 is turned off when a door enclosing the tank 38 and/or reservoir 44 is open.

The controller 48 may include processing circuitry 52 configured to operate and control the various functions of the cryoablation console 12 and the cryoablation catheter 16. The cryoablation catheter 16 may provide for diagnostic, energetic, therapeutic and/or investigatory interaction between the cryoablation catheter 16 and a treatment site. The treatment may deliver, for example, the pressurized refrigerant to the inflatable portion 20 of the cryoablation catheter 16 sufficient to cryoablate a tissue area.

In one or more embodiments, the processing circuitry 52 may include a memory 54 and a processor 56. In particular, in addition to or instead of a processor, such as a central processing unit, and memory, the processing circuitry 52 may comprise integrated circuitry for processing and/or control, e.g., one or more processors and/or processor cores and/or FPGAs (Field Programmable Gate Array) and/or ASICs (Application Specific Integrated Circuitry) adapted to execute instructions. The processor 56 may be configured to access (e.g., write to and/or read from) the memory 54, which may comprise any kind of volatile and/or nonvolatile memory, e.g., cache and/or buffer memory and/or RAM (Random Access Memory) and/or ROM (Read-Only Memory) and/or optical memory and/or EPROM (Erasable Programmable Read-Only Memory).

The processing circuitry 52 may be configured to control any of the methods and/or processes described herein and/or to cause such methods, and/or processes to be performed, e.g., by controller 48. Processor 56 corresponds to one or more processors for performing functions described herein. The memory 54 is configured to store data, programmatic software code and/or other information described herein. In some embodiments, the software may include instructions that, when executed by the processor 56 and/or processing circuitry 52 causes the processor 56 and/or processing circuitry 52 to perform the processes described herein with respect to controller 48. For example, processing circuitry 52 of the controller 48 may be in communication with an input device 62 that is configured to enable an operator to operate the cryoablation system 10 and input values such as target pressure to control the delivery of pressurized refrigerant to the cryoablation catheter 16. The input device 62 may include a keyboard and mouse. A display device 64 may display system parameters such as a temperature and pressure of the pressurized refrigerant in the reservoir 44, as well as a display of whether the heater 46 is on or off. Thus, in some embodiments, a cryoablation system 10 includes a tank 38 containing pressurized refrigerant and a reservoir 44 between the tank 38 and the cryoablation catheter 16 and configured to store pressurized refrigerant received from the tank 38.

According to aspects of this disclosure, the cryoablation system 10 also includes a heater 46 configured to heat the stored pressurized refrigerant to achieve a target pressure, and a cryoablation catheter 16 configured to receive the heated pressurized refrigerant. In some examples, heating provides an advantage of providing more rapid arrival at a desired pressure over previous systems without heating.

When pressurized refrigerant is injected into the treatment portion 18 via the elongate body 28, the treatment portion 18 may expand, causing the electrodes 40 to contact surrounding tissue and sense electrical activity in the tissue in the vicinity of the electrodes 40. The electrical activity sensed by the electrodes 40 may be conducted by wires to the controller 48 that records in the memory 54 and displays on display device 64 the electrical activity sensed by the cryoablation catheter 16. In some embodiments, the controller 48 may include other equipment such as an electrocardiograph (ECG). The signals that are displayed on the display device 64 may assist the medical provider in determining, among other things, an amount of electrical activity of the tissue and how well the treatment portion 18 is making contact with the surrounding tissue and whether the ablation and/or cryoablation in a current position is likely to succeed or whether the treatment portion 18 should be repositioned and a new mapping obtained.

In addition to, or in the alternative to the treatment portion 18, the distal end 34 of the elongate body 28 may be equipped with a thermal tip 36 that removes heat from the tissue to reach a first temperature for cryo-mapping and to reach a second temperature that ablates the tissue. The target temperature for the thermal tip 36 is higher for cryo-mapping than for cryoablation, so that for cryo-mapping the cellular effects are reversible and for cryoablation the cellular effects are irreversible.

Pressurized refrigerant may be provided by the tank 38 within the cryoablation console 12. The coolant may be N₂O or another type of cooling gas and/or liquid may pass through internal piping of the cryoablation console 12 to the reservoir 44 before being transferred to the cryoablation catheter 16 via the coaxial cable umbilical 22. At the distal end of the coaxial cable umbilical 22, inside the cryoablation catheter 16, the pressurized refrigerant may be released inside the catheter tip cavity, which is under vacuum. Both the phase change from liquid to gas and the sudden expansion of the pressurized refrigerant are endothermic reactions, causing a temperature differential which may result in the catheter tip or balloon freezing. The pressurized refrigerant vapor may then be returned through the vacuum path of the umbilical system 14 and into the cryoablation console 12, where it may be evacuated.

The processor 56 may be configured to provide temperature control 58 to set a temperature of the pressurized refrigerant in reservoir 44 via heating element 46. Processor 56 may also be configured to compare the temperature of the pressurized refrigerant in reservoir 44 measured by a thermocouple of the sensors 50 to a target temperature that may be input by the operator via the input device 62.

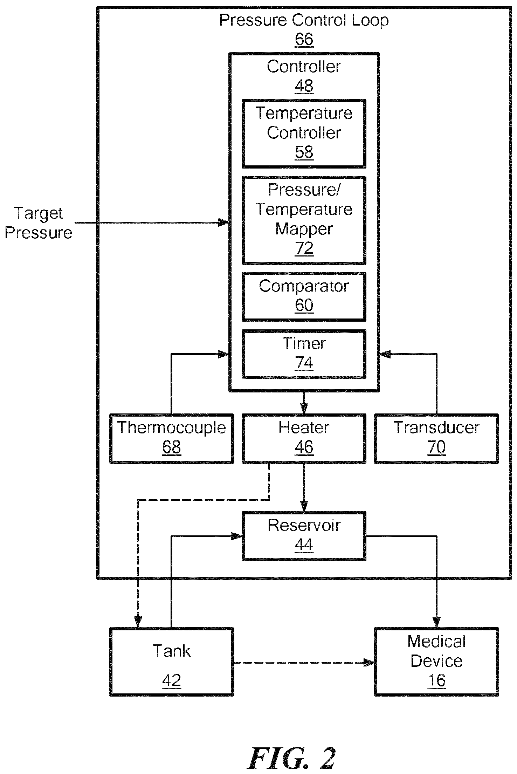

FIG. 2 is a block diagram of a pressure control loop 66 implemented using the controller 48, the heater 46, the tank 38 and/or reservoir 44, and at least one of a thermocouple 68 and a pressure transducer 70, collectively referred to as sensors 50. In addition to the temperature controller 58 and the comparator 60, the controller 48 may also include a pressure/temperature mapper 72, which maps a target pressure to a target temperature. The pressure/temperature mapper 72 may also be configured to map a pressure measured by the pressure transducer 70 to a reservoir temperature. In the alternatively, the thermocouple 68 may measure reservoir temperature directly. The comparator 60 may be configured to determine a difference between the target temperature and the reservoir temperature to produce a temperature error signal.

The temperature controller 58 may be configured to adjust a temperature setting of the heater 46 to increase or decrease heat output of the heater 46 to drive the temperature error signal toward zero. In the alternative, the comparator 60 may be configured to determine a difference between the target pressure and the pressure measured by the pressure transducer 70 to determine a pressure error. The determined pressure error may then be mapped to a new temperature setting by the pressure/temperature mapper 72. This new temperature setting may be used by the temperature controller 58 to set a heat output of the heater 46 to achieve the target pressure. Note that in some embodiments, the temperature controller 58 may also be responsive to an environmental temperature inside and/or outside the cryoablation console 12 to raise or lower the temperature of the pressurized refrigerant in the tank 38 and/or reservoir 44.

Thus, in some embodiments, the cryoablation system 10 includes a pressure transducer 70 configured to detect a pressure of the pressurized refrigerant received by the cryoablation catheter 16. The cryoablation system 10 may also include a thermocouple 68 configured to detect a temperature of the pressurized refrigerant in the tank 38 and/or reservoir 44. The cryoablation system 10 also includes a controller 48 to control a temperature setting of the heater 46 based at least in part on at least one of the detected pressure and the detected temperature. The controller 48 is configured to drive a difference between the detected pressure and a target pressure toward zero. Note that although the heater 46, thermocouple 68 and pressure transducer 70 are shown exterior to the tank 38 and/or reservoir 44, any one or more of these components can be included in the interior of the tank 38 and/or reservoir 44.

In some embodiments, a cryoablation console 12 is configured to controllably deliver a pressurized refrigerant to a cryoablation catheter 16. The cryoablation console 12 includes a tank 38 and/or a reservoir 44 configured to store pressurized refrigerant from the tank 38. The cryoablation console 12 includes a heating element 46 configured to heat the pressurized refrigerant and a pressure transducer 70 configured to detect a pressure of the pressurized refrigerant. The cryoablation console 12 further includes a controller 48 configured to set a temperature of the heating element 46 to drive the detected pressure toward a target pressure.

In some embodiments, the cryoablation console 12 also includes a graphical user interface (GUI), which includes the input device 62 operating in conjunction with the display device, 64, configured to enable an operator of the cryoablation console 12 to set the target pressure. In some embodiments, controller 48 is further configured to cycle through a sequence of temperature settings based at least in part on a plurality of target pressure settings input by the operator using the GUI 62, 64. In some embodiments, the sequence of temperature settings are determined to deliver cryotherapy at a target pressure during successive time intervals. In some embodiments, the cryoablation console 12 includes a timer 74 configured to limit a time duration of an on state of the heating element 46 to override the temperature setting of the controller 48. In some embodiments, the cryoablation console 12 further includes a sensor 50 configured to sense removal of the tank 38 from the cryoablation console 12, and wherein the controller 48 is further configured to turn off the heating element 46 when the tank 38 is removed from the cryoablation console 12. In some embodiments, the controller 48 is configured to limit the temperature setting of the heating element 46 to maintain the detected pressure below a threshold. In some embodiments, the cryoablation console 12 also includes a thermocouple 68 configured to measure a temperature of the pressurized refrigerant. In some embodiments in which the reservoir is heated and is removable, the controller may be configured to turn off the heating element when the reservoir is removed.

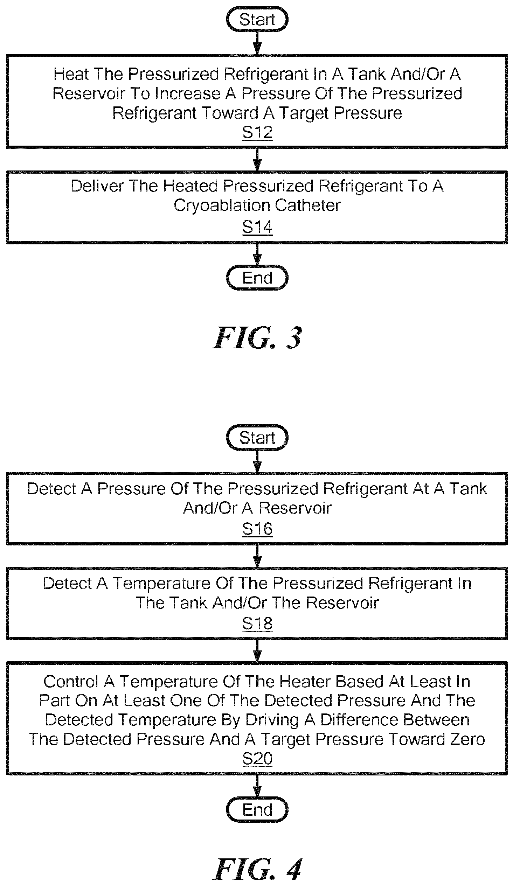

FIG. 3 is a flowchart of an example process in a cryoablation system 10 for managing pressure of a pressurized refrigerant by heating a at least one of a reservoir and a tank of the pressurized refrigerant. The process may be performed by the controller 48 including the temperature controller 58, pressure/temperature mapper 72 and comparator 60, as well as the heater 46. The process includes heating the pressurized refrigerant in at least one of the reservoir 44 and the tank 38 to increase a pressure of the pressurized refrigerant toward a target pressure (Block S12). The process further includes delivering the heated pressurized refrigerant to a cryoablation catheter 16 (Block S14).

In some embodiments, the process also includes measuring a pressure of the pressurized refrigerant and adjusting a temperature of the pressurized refrigerant to drive the measured pressure toward the target pressure. In some embodiments, the heating is applied in cycles to successively increase and decrease the pressure of the pressurized refrigerant according to input from an operator of the cryoablation system 10 and/or in accordance with the execution of software by the processor 56, for example. In some embodiments, the process also includes ceasing heating the pressurized refrigerant when the pressure reaches an upper limit. In some embodiments, the process also includes ceasing heating the pressurized refrigerant when the tank 38 is removed. In some embodiments, detection of removal of the tank 38 may be by a sensor 50 such as an electro-mechanical switch. In some embodiments, the reservoir 44 is unremovable, and in some embodiments, the tank 38 is removable. In some embodiments, the reservoir 44 is removable.

FIG. 4 is a flowchart of a process for controlling pressure of a pressurized refrigerant. The process may be performed by the controller 48, thermocouple 68 and pressure transducer 70. The process includes detecting, via the pressure transducer 70, a pressure of the pressurized refrigerant in the tank 38 and/or the reservoir 44 (Block S 16). The process also includes detecting, via the thermocouple 68, a temperature of the pressurized refrigerant in the tank 38 and/or reservoir 44 (Block S18). The process also includes controlling, via the temperature controller 58, a temperature of the heater based at least in part on at least one of the detected pressure and the detected temperature, by driving a difference between the detected pressure and a target pressure toward zero (Block S20). The difference between the detected pressure and the target pressure may be determined by the comparator 60.

In some embodiments, the process may also include timing, via of a timer 74 of the controller 48, a duration of at least one of an on state and an off state of the heater 46. The timer 74 may include circuitry to prevent the heater from heating the pressurized refrigerant when there is a malfunction of the thermocouple 68 and/or the pressure transducer 70 or when a valve of the reservoir 44 is not open. In some embodiments, the process may further include limiting, via the temperature controller 58, the temperature of the heater 46 to maintain a pressure of the pressurized refrigerant in the reservoir 44 below a threshold. The process may also include detecting, via a sensor 50, removal of the reservoir 44, and turning the heater 46 off when removal of the reservoir 44 is detected.

In one or more examples, the described techniques may be implemented in hardware, software, firmware, or any combination thereof. If implemented in software, the functions may be stored as one or more instructions or code on a computer-readable medium and executed by a hardware-based processing unit. Computer-readable media may include non-transitory computer-readable media, which corresponds to a tangible medium such as data storage media (e.g., RAM, ROM, EEPROM, flash memory, or any other medium that can be used to store desired program code in the form of instructions or data structures and that can be accessed by a computer).

Instructions may be executed by one or more processors, such as one or more digital signal processors (DSPs), general purpose microprocessors, application specific integrated circuits (ASICs), field programmable logic arrays (FPGAs), or other equivalent integrated or discrete logic circuitry. Accordingly, the term "processor" as used herein may refer to any of the foregoing structure or any other physical structure suitable for implementation of the described techniques. Also, the techniques could be fully implemented in one or more circuits or logic elements.

It will be appreciated by persons skilled in the art that the present invention is not limited to what has been particularly shown and described herein above. In addition, unless mention was made above to the contrary, it should be noted that all of the accompanying drawings are not to scale. A variety of modifications and variations are possible in light of the above teachings without departing from the scope of the following claims.

Further disclosed herein is the subject-matter of the following clauses:
1. A cryoablation system configured to provide cryotherapy by delivering a pressurized refrigerant to a cryoablation catheter, the cryoablation system comprising:
   at least one of a reservoir and a tank configured to store pressurized refrigerant;
   a heater configured to heat the stored pressurized refrigerant to drive a pressure of delivery of the pressurized refrigerant toward a target pressure; and
   a cryoablation catheter configured to receive the heated pressurized refrigerant.
2. The cryoablation system of Clause 1, wherein the heater is removably affixed to at least one of the at least one of a reservoir and a tank.
3. The cryoablation system of Clause 1 or of any of the preceding Clauses, further comprising a console configured to house the at least one of a reservoir and a tank and the heater, the console providing a graphical user interface (GUI) configured to generate a graphical indication of a status of the heater.
4. The cryoablation system of Clause 1 or of any of the preceding Clauses,
   wherein the heater is configured to have an upper temperature limit such that the heater does not heat the stored pressurized refrigerant beyond the upper temperature limit.
5. The cryoablation system of Clause 1 or of any of the preceding Clauses, further comprising:
   a pressure transducer configured to detect a pressure of the pressurized refrigerant in at least one of the at least one of a reservoir and a tank;
   a thermocouple configured to detect a temperature of the pressurized refrigerant in the at least one of the at least one of a reservoir and a tank; and
   a controller to control a temperature of the heater based at least in part on at least one of the detected pressure and the detected temperature, the controller configured to drive the detected pressure toward a target pressure.
6. The cryoablation system of Clause 5, wherein the controller includes a timer to time a duration of at least one of an on state and an off state of the heater.
7. The cryoablation system of Clause 5 or of any of Clauses 5-6, wherein the controller is configured to limit the temperature of the heater to maintain a pressure of the pressurized refrigerant in at least one of a reservoir and a tank below a threshold.
8. The cryoablation system of Clause 5 or of any of Clauses 5-7, wherein the controller is further configured to detect removal of the tank and to turn the heater off when removal of the tank is detected.
9. A cryoablation console configured to controllably deliver a pressurized refrigerant to a cryoablation catheter, the cryoablation console comprising:
   at least one of a reservoir and a tank configured to store pressurized refrigerant;
   a heating element configured to heat the pressurized refrigerant;
   a pressure transducer configured to detect a pressure of the pressurized refrigerant; and
   a controller configured to control a temperature of the heating element to drive the detected pressure toward a target pressure.
10. The cryoablation console of Clause 9, wherein the heating element is removably affixed to at least one of the at least one of a reservoir and a tank.
11. The cryoablation console of Clause 9 or of any of Clauses 9-10, further comprising a graphical user interface (GUI) configured to generate a graphical indication of a status of the heater.
12. The cryoablation console of Clause 9 or of any of Clauses 9-11 wherein a sequence of temperature settings are determined to deliver cryotherapy at a sequence of target refrigerant pressures during successive time intervals.
13. The cryoablation console of Clause 9 or of any of Clauses 9-12, further comprising a timer configured to time an on state of the heating element, and wherein the controller is configured to limit heating of the pressurized refrigerant based on the timer.
14. The cryoablation console of Clause 9 or of any of Clauses 9-13, further comprising a sensor configured to sense removal of the tank from the cryoablation console, and wherein the controller is further configured to turn off the heating element when the tank is removed from the cryoablation console.
15. The cryoablation console of Clause 9 or of any of Clauses 9-14, wherein the controller is configured to adjust the temperature of the heating element to maintain the detected pressure below a threshold.
16. The cryoablation console of Clause 9 or of any of Clauses 9-15, further comprising a thermocouple configured to measure a temperature of the pressurized refrigerant.
17. A method of increasing pressure of a pressurized refrigerant in a cryoablation system, the method comprising:
   heating, based on a target pressure, the pressurized refrigerant in at least one of a reservoir and a tank to increase a pressure of the pressurized refrigerant; and
   delivering the heated pressurized refrigerant to a cryoablation catheter.
18. The method of Clause 17, further comprising measuring a pressure of the pressurized refrigerant and adjusting a temperature of the pressurized refrigerant to drive he measured pressure toward the target pressure.
19. The method of Clause 17 or of any of Clauses 17-18, further comprising ceasing heating the pressurized refrigerant when the pressure reaches an upper limit.
20. The method of Clause 17 or of any of Clauses 17-19, further comprising ceasing heating the pressurized refrigerant when the reservoir is removed.

## Claims

1. A cryoablation system configured to provide cryotherapy by delivering a pressurized refrigerant to a cryoablation catheter, the cryoablation system comprising:
at least one of a reservoir and a tank configured to store pressurized refrigerant;
a heater configured to heat the stored pressurized refrigerant to drive a pressure of delivery of the pressurized refrigerant toward a target pressure; and
a cryoablation catheter configured to receive the heated pressurized refrigerant.

2. The cryoablation system of Claim 1, wherein the heater is removably affixed to at least one of the at least one of a reservoir and a tank.

3. The cryoablation system of any of Claims 1-2, further comprising a console configured to house the at least one of a reservoir and a tank and the heater, the console providing a graphical user interface (GUI) configured to generate a graphical indication of a status of the heater, and/or wherein the heater is configured to have an upper temperature limit such that the heater does not heat the stored pressurized refrigerant beyond the upper temperature limit.

4. The cryoablation system of any of Claims 1-3, further comprising:
a pressure transducer configured to detect a pressure of the pressurized refrigerant in at least one of the at least one of a reservoir and a tank;
a thermocouple configured to detect a temperature of the pressurized refrigerant in the at least one of the at least one of a reservoir and a tank; and
a controller to control a temperature of the heater based at least in part on at least one of the detected pressure and the detected temperature, the controller configured to drive the detected pressure toward a target pressure.

5. The cryoablation system of Claim 4, wherein the controller includes a timer to time a duration of at least one of an on state and an off state of the heater.

6. The cryoablation system of any of Claims 4-5, wherein the controller is configured to limit the temperature of the heater to maintain a pressure of the pressurized refrigerant in at least one of a reservoir and a tank below a threshold, and/or wherein the controller is further configured to detect removal of the tank and to turn the heater off when removal of the tank is detected.

7. A cryoablation console configured to controllably deliver a pressurized refrigerant to a cryoablation catheter, the cryoablation console comprising:
at least one of a reservoir and a tank configured to store pressurized refrigerant;
a heating element configured to heat the pressurized refrigerant;
a pressure transducer configured to detect a pressure of the pressurized refrigerant; and
a controller configured to control a temperature of the heating element to drive the detected pressure toward a target pressure.

8. The cryoablation console of Claim 7, wherein the heating element is removably affixed to at least one of the at least one of a reservoir and a tank.

9. The cryoablation console of any of Claims 7-8, further comprising a graphical user interface (GUI) configured to generate a graphical indication of a status of the heater.

10. The cryoablation console of any of Claims 7-9 wherein a sequence of temperature settings are determined to deliver cryotherapy at a sequence of target refrigerant pressures during successive time intervals.

11. The cryoablation console of any of Claims 7-10, further comprising a timer configured to time an on state of the heating element, and wherein the controller is configured to limit heating of the pressurized refrigerant based on the timer, and/or further comprising a sensor configured to sense removal of the tank from the cryoablation console, and wherein the controller is further configured to turn off the heating element when the tank is removed from the cryoablation console.

12. The cryoablation console of any of Claims 7-11, wherein the controller is configured to adjust the temperature of the heating element to maintain the detected pressure below a threshold, and/or further comprising a thermocouple configured to measure a temperature of the pressurized refrigerant.

13. A method of increasing pressure of a pressurized refrigerant in a cryoablation system, the method comprising:
heating, based on a target pressure, the pressurized refrigerant in at least one of a reservoir and a tank to increase a pressure of the pressurized refrigerant; and
delivering the heated pressurized refrigerant to a cryoablation catheter.

14. The method of Claim 13, further comprising measuring a pressure of the pressurized refrigerant and adjusting a temperature of the pressurized refrigerant to drive he measured pressure toward the target pressure.

15. The method of any of Claims 13-14, further comprising ceasing heating the pressurized refrigerant when the pressure reaches an upper limit, and/or further comprising ceasing heating the pressurized refrigerant when the reservoir is removed.
